# EUROPÄISCHE PATENTSCHRIFT

(11) **EP 1 613 225 B1**
(45) Veröffentlichungstag und Bekanntmachung des Hinweises auf die Patenterteilung: **10.09.2008**
(21) Anmeldenummer: 03709556.9
(22) Anmeldetag: 15.04.2003
(51) Int. Cl.: A61B 17/70

(54) **VORRICHTUNG ZUR KNOCHENFIXATION**
BONE FIXATION DEVICE
DISPOSITIF DE FIXATION D'OS

(43) Veröffentlichungstag der Anmeldung: 11.01.2006
(73) Patentinhaber: Synthes GmbH, 4436 Oberdorf (CH)
(72) Erfinder: FRIGG, Robert, CH-2544 Bettlach (CH); LÄNG, Bruno, CH-4557 Horriwil (CH)
(74) Vertreter: Lusuardi, Werther
(86) Internationale Anmeldenummer: PCT/CH2003/000253
(87) Internationale Veröffentlichungsnummer: WO 2004/091413

(56) Entgegenhaltungen:
- EP-A- 0 528 706
- DE-A- 10 117 426
- FR-A- 2 734 471
- FR-A- 2 745 707
- US-A- 5 282 863
- US-B1- 6 471 705

## Beschreibung

Die Erfindung bezieht sich auf Vorrichtung zur Knochenfixation gemäss dem Oberbegriff des Patentanspruchs 1.

Bei den am meisten verwendeten, bekannten Wirbelsäulen-Stabilisierungssystemen werden steife Verbindungsstäbe verwendet, um die an der Wirbelsäule verankerten Elemente zu verbinden. Diese steifen Verbindungen erlauben es, die vom Chirurgen durchgeführten Korrekturen der Wirbelsäue in einer gewünschten Lage zu fixieren. Der Nachteil solcher steifer Verbindungen ist deren intraoperative Handhabung. Ausser in Ausnahmefällen gelingt es dem Chirurgen nicht, mehrere Verankerungselemente derart an der Wirbelsäule anzubringen, dass diese mit einem geraden steifen Stab verbunden werden können. Aus diesem Grund muss der rigide Stab intraoperativ angeformt werden. Dieses Anformen ist aufwendig und kann zu ungewollten Stress-Konzentrationen an den Verankerungselementen führen. Um die intraoperative Handhabung dieser Systeme zu verbessern, wurden deshalb Verankerungselemente entwickelt, deren Stabverbindungselemente polyaxial schwenkbar ausgestaltet sind. Diese Schwenkbarkeit erleichtert zwar das Einführen des steifen Stabes, da dieser nicht mehr so exakt vorgeformt werden muss, dafür besteht aber nun der Nachteil des massiven Volumens solcher polyaxialer Verbindungselemente. Unabhängig davon, ob es sich somit um einfache oder um polyaxial verschwenkbare Verankerungselemente handelt, besteht der grosse Nachteil, dass bei erfolgter Verbindung mit einem steifen Stab eine zusätzliche Korrektur der Wirbelsäule nur noch sehr eingeschränkt möglich ist.

Aus der US-A 3,997,138 CROCK ET AL. ist eine Knochenfixationsvorrichtung für die Wirbelsäule bekannt, bei welcher zwei Pedikelschrauben in die betroffenen, benachbarten Wirbelkörper implantiert und mittels zweier in den Köpfen der Pedikelschrauben in geschlossenen Kanälen einklemmbaren Stäben untereinander verbunden werden. Nachteilig bei dieser Vorrichtung ist der Umstand, dass bei Verwendung von flexiblen Stäben die Gesamtvorrichtung nach erfolgter Fixation immer noch flexibel bleibt und nicht wirklich versteift wird.

Eine weitere Knochenfixationsvorrichtung für die Wirbelsäule ist aus der DE 101 17 426 bekannt.

Aus der US-A 4,041,939 HALL ist eine weitere Knochenfixationsvorrichtung für die Wirbelsäule bekannt, bei welcher eine Serie von Pedikelschrauben in die Wirbelkörper des betroffenen Wirbelsäulensegmentes implantiert werden und mittels eines in den Köpfen der Pedikelschrauben in geschlossenen Kanälen einklemmbaren Kabels untereinander verbunden werden. Diese Vorrichtung weist somit den gleichen Nachteil wie CROCK ET AL. auf, weil auch hier nach erfolgter Fixation des Kabels in den Köpfen der Pedikelschrauben die Gesamtvorrichtung immer noch flexibel bleibt und keine Versteifung erzielt wird.

Die bekannten flexiblen Verbindungselemente in Form von dünnen Drähten, dünnen Stäben oder speziellen, flexiblen Kabeln zwischen den Verankerungselementen haben somit alle den Nachteil, dass sie eine vom Chirurgen durchgeführte Korrektur, ausser in Zugrichtung, nicht ausreichend fixieren können. Eine Entlastung der Wirbelsäule, eine Lagekorrektur oder eine Frakturstabilisierung kann deshalb mittels der bekannten, flexiblen Stabsysteme nicht erfolgen.

Hier will die Erfindung Abhilfe schaffen. Der Erfindung liegt die Aufgabe zugrunde, eine Vorrichtung zur Knochenfixation zu schaffen, welche einerseits eine grosse Flexibilität an die anatomischen Verhältnisse und die chirurgisch erzielten Resultate während der Implantation der Vorrichtung erlaubt, anderseits durch eine einfache Blockierung der gegeneinander beweglichen Elemente der Vorrichtung deren vollständige Versteifung erlaubt. Zudem soll eine minimal invasive Behandlung der Wirbelsäule ermöglicht werden.

Die Erfindung löst die gestellte Aufgabe mit einer Vorrichtung, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung das umständliche Anpassen von steifen Längsträgern entfällt ohne aber die Nachteile von flexiblen Längsträgern in Kauf nehmen zu müssen. Zudem kann nach erfolgreicher Verbindung der einzelnen Knochenfixationselemente an der Wirbelsäule die Position der einzelnen Knochenfixationselemente, relativ zueinander, geändert werden. Diese neue Position der betroffenen Wirbelkörper zueinander (Wirbelsäulen-Korrektur) kann durch Blockieren der flexiblen Verbindungselemente in den Knochenfixationselemente fixiert werden. Dank der Elastizität der flexiblen Verbindungselemente, ist ihre Einführung durch einen minimalen Weichteilzugang möglich.

Bei einer besonderen Ausführungsform weisen die Knochenfixationselemente ein Blockierelement auf, mit welchem die Beweglichkeit von in ihrem Durchgang eingeführten Verbindungselementen relativ zum Knochenfixationselement wahlweise fixierbar und lösbar ist. Durch eine solche Blockierung der flexiblen Verbindungselemente in den Durchgängen zweier benachbarter Knochenfixationselemente wird ihre Verschiebbarkeit in den Durchgängen aufgehoben, wobei eine Versteifung der gesamten Vorrichtung resultiert.

Die Knochenfixationselemente können aus folgender Gruppe ausgewählt werden: Knochenschrauben, Knochenhaken und Knochenplatten.

Bei einer besonderen Ausführungsform ist Durchgang ein peripher geschlossener Kanal. Der Vorteil eines geschlossenen Kanals liegt in der Möglichkeit, die Dimension der Knochenfixationselemente zu reduzieren und deren Design zu vereinfachen. Typischerweise ist der Durchgang ein peripher offener Kanal, vorzugsweise in Form einer Nut mit U-förmigem Profil. Das U-förmige Profil hat den Vorteil, dass die flexiblen Verbindungselemente über die U-förmige Öffnung darin eingesetzt werden können und nicht eingefädelt werden müssen. Zudem besteht die Möglichkeit - nach erfolgter Montage der flexiblen Verbindungselemente - ein zusätzliches Knochenfixationselement sekundär einzubringen.

Bei einer besonderen Ausführungsform liegen die Verbindungselemente in Form von Stäben, vorzugsweise mit einem runden Querschnittsprofil vor.

Die Anzahl der Verbindungselemente beträgt vorzugsweise vier, fünf, sechs oder sieben.

Bei einer besonderen Ausführungsform ist der Querschnitt der Durchgänge und der Querschnitt der Verbindungselemente derart aufeinander abgestimmt, dass die Verbindungselemente formschlüssig in den Durchgängen aufnehmbar sind.
Der Querschnitt der Durchgänge ist zweckmässig polyedrisch, vorzugsweise quadratisch, rechteckig oder dreieckig ausgebildet. Die polyedrische Ausbildung des Durchgangsprofils hat den Vorteil, dass die Verbindungselemente in eine definierte Position zu liegen kommen. Somit kann die maximale Biegesteifigkeit der Verbindung zweier Verbindungselemente, beziehungsweise der gesamten Vorrichtung in einer Richtung vordefiniert werden.

Bei einer besonderen Ausführungsform berühren sich die Verbindungselemente im Durchgang gegenseitig. Durch diese Massnahme wird bei der Blockierung die Längsverschieblichkeit und die Rotation der Verbindungselemente zueinander aufgehoben, was zur erwünschten Versteifung der gesamten Vorrichtung führt.

Bei einer weiteren Ausführungsform sind die Knochenfixationselemente als Knochenschrauben ausgebildet und der im Knochen verankerbare Teil ist ein gewindeter Schraubenschaft, wobei die Knochenschraube einen Kopf aufweist in welchem ein Durchgang in Form eines U-förmigen Schlitzes realisiert ist, der mit einem als Kappe realisierten Blockierelement derart verschliessbar ist, dass die Verschieblichkeit von sich im Schlitz befindlichen Verbindungselementen blockierbar ist.

Bei einer besonderen Ausführungsform weist die Oberfläche der flexiblen Verbindungselemente eine Aufrauhung oder Mikrostrukturierung auf. Dadurch kann eine Erhöhung der Reibhaftung der Verbindungselemente untereinander erzielt werden.

Bei einer weiteren Ausführungsform weist die Oberfläche der flexiblen Verbindungselemente eine Makrostrukturierung auf. Durch diese Massnahme wird ebenfalls eine Erhöhung der Reibhaftung der Verbindungselemente untereinander erreicht.

Bei einer weiteren Ausführungsform weist die Oberfläche des Durchgangs eine Aufrauhung oder Mikrostrukturierung auf. Dadurch ergibt sich eine Erhöhung der Reibhaftung gegenüber den Verbindungselementen.

Nachstehend wird zum besseren Verständnis der Erfindung noch ein Verfahren zur Knochenfixation offenbart, welches aus folgenden Operationsschritten besteht:
a) Je ein Knochenfixationselement mit einem Durchgang wird an oder in einem Knochen oder Knochenfragment mittels seines verankerbaren Teils verankert;
b) zwei oder mehrere longitudinale, flexible Verbindungselemente werden in die Durchgänge der beiden benachbarten Knochenfixationselemente eingeführt; und
c) die Durchgänge der beiden benachbarten Knochenfixationselemente werden mit einem Blockierelement verschlossen, wobei die Beweglichkeit der im Durchgang eingeführten Verbindungselementen relativ zum Knochenfixationselement blockiert wird und dabei die gesamte Vorrichtung versteift wird.

Die mit diesem Verfahren erzielbaren Vorteile sind vielfältig:
(1) Allfällige Abweichungen von der optimalen Position der beiden Knochenfixationselemente spielen keine Rolle, da die flexiblen Verbindungselemente eine grosse Variabilität zulassen (im Vergleich zu den starren Längsträgern gemäss dem Stand der Technik, die in einem solchen Fall zuerst aus dem Patienten entfernt und vom Chirurgen mit einem Spezialgerät zurechtgebogen werden müssen);
(2) durch die Blockierung der Verbindungselemente wird ihre Flexibilität aufgehoben und es resultiert eine Versteifung der gesamten Vorrichtung, so dass der Endzustand gleich ist, wie bei Verwendung eines steifen Längsträgers; und
(3) die Position der einzelnen Knochenfixationselemente kann bei liegenden Verbindungselemente, relativ zueinander, geändert und in der neuen Position fixiert werden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer erfindungsgemässen Vorrichtung mit zwei Pedikelschrauben und vier flexiblen Längsstäben im nicht-blockierten, flexiblen Zustand;
Fig. 2 einen Querschnitt durch den Kopf einer Pedikelschraube der Vorrichtung nach Fig. 1; und
Fig. 3 einen Querschnitt durch eine Variante eines Kopfes einer Pedikelschraube mit geschlossenem Durchgang.

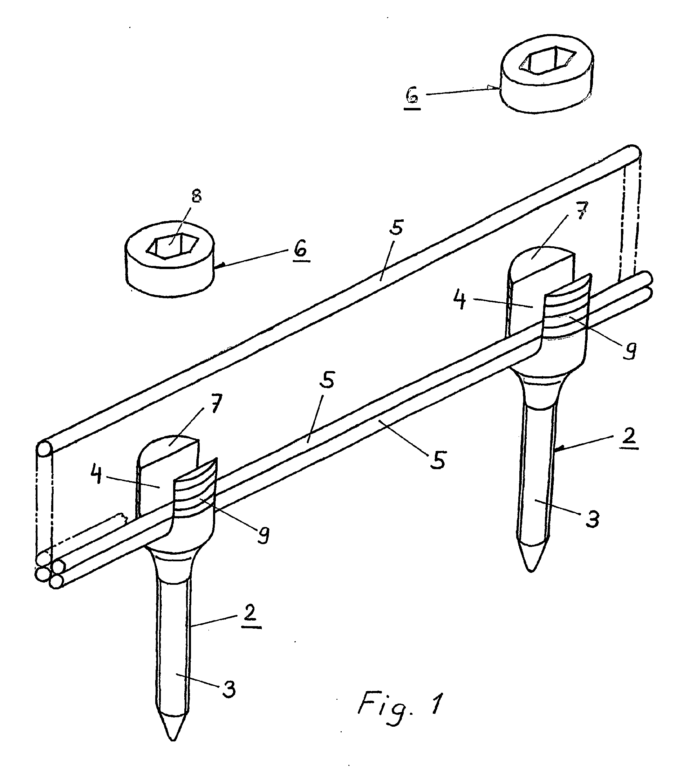

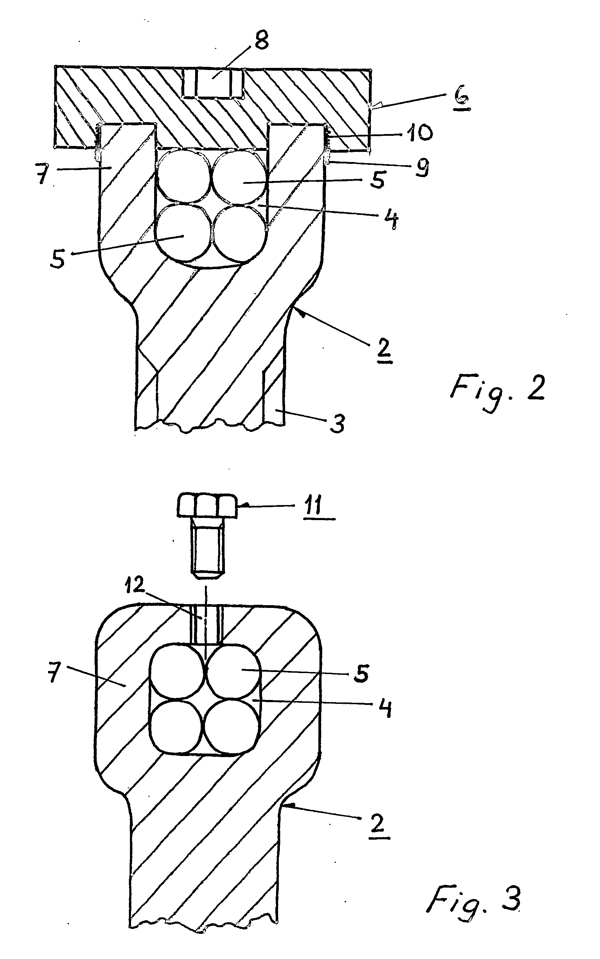

Die in Fig. 1 dargestellte Vorrichtung zur Knochenfixation 1 umfasst zwei Knochenfixationselemente 2 in Form von Pedikelschrauben sowie vier longitudinale, flexible Verbindungselemente 5, in Form von dünnen, flexiblen Rundstäben welche mit den Knochenfixationselementen 2 verbindbar sind. Die Knochenfixationselemente 2 weisen zu diesem Zweck einen im Knochen verankerbaren Teil 3 in Form eines gewindeten Schaftes sowie einen Kopf 7 mit einem U-förmigen Durchgang 4 auf. Die Durchgänge 4 der Knochenfixationselemente 2 sind mit einem Blockierelement 6 in Form einer hohlen Kappe 6 mit einem Innengewinde 10 (Fig. 2) und einem Innensechskant 8 verschliessbar. Zu diesem Zweck weist der Kopf 7 der Knochenfixationselemente 2 ein Aussengewinde 9 auf, auf welches das Blockierelement 6 aufgeschraubt werden kann, derart dass die im Durchgang 4 befindlichen longitudinalen, flexiblen Verbindungselemente 5 sowohl untereinander als auch relativ zum betreffenden Knochenfixationselement 2 in ihrer Position fixiert werden. Dieser fixierte Zustand der Vorrichtung 1 ist in Fig. 2 dargestellt.

In Fig. 3 ist eine Variante eines Knochenfixationselementes 2 dargestellt, bei welchem statt eines offenen, U-förmigen Durchgangs 4 ein geschlossener, kanalförmiger Durchgang 4 mit annähernd quadratischem Profil vorliegt. Um die im Durchgang 4 eingelegten Verbindungselemente 5 zu fixieren, ist dazu eine Fixationsschraube 11 vorgesehen, welche durch die in den Durchgang 4 reichende Bohrung 11 eingeschraubt werden kann, bis sie auf die Verbindungselemente 5 drückt und dadurch deren Blockierung bewirkt.

## Patentansprüche

1. Vorrichtung zur Knochenfixation (1) mit mindestens zwei Knochenfixationselementen (2), welche beide einen am oder im Knochen verankerbaren Teil (3) und einen Durchgang (4) aufweisen, wobei die Vorrichtung (1) mindestens zwei, in die Durchgänge (4) von jeweils zwei benachbarten Knochenfixationselementen (2) einführbare und darin befestigbare, longitudinale, flexible Verbindungselemente (5) umfasst.
**dadurch gekennzeichnet, dass**
die Anzahl der Verbindungselemente (5) vier, fünf, sechs oder sieben beträgt.

2. Vorrichtung (1) nach Anspruch 1, **dadurch gekennzeichnet, dass** die Knochenfixationselemente (2) ein Blockierelement (6) aufweisen, mit welchem die Beweglichkeit von im Durchgang (4) eingeführten Verbindungselementen (5) relativ zum Knochenfixationselement (2) wahlweise fixierbar und lösbar ist.

3. Vorrichtung (1) nach Anspruch 1 oder 2, **dadurch gekennzeichnet, dass** die Knochenfixationselemente (2) aus folgender Gruppe ausgewählt sind: Knochenschrauben, Knochenhaken und Knochenplatten.

4. Vorrichtung (1) nach einem der Ansprüche 1-3, **dadurch gekennzeichnet, dass** der Durchgang (4) ein peripher geschlossener Kanal ist.

5. Vorrichtung (1) nach einem der Ansprüche 1-3, **dadurch gekennzeichnet, dass** der Durchgang (4) ein peripher offener Kanal ist, vorzugsweise in Form einer Nut mit U-förmigem Profil.

6. Vorrichtung (1) nach einem der Ansprüche 1-5, **dadurch gekennzeichnet, dass** die Verbindungselemente (5) in Form von Stäben, vorzugsweise mit einem runden Querschnittsprofil vorliegen.

7. Vorrichtung (1) nach einem der Ansprüche 1-6, **dadurch gekennzeichnet, dass** der Querschnitt der Durchgänge (4) und die Querschnitte der Verbindungselemente (5) derart aufeinander abgestimmt sind, dass die Verbindungselemente (5) formschlüssig in den Durchgängen (4) aufnehmbar sind.

8. Vorrichtung (1) nach einem der Ansprüche 1-7, **dadurch gekennzeichnet, dass** der Querschnitt der Durchgänge (4) polyedrisch, vorzugsweise quadratisch, rechteckig oder dreieckig ausgebildet ist.

9. Vorrichtung (1) nach einem der Ansprüche 1-8, **dadurch gekennzeichnet, dass** sich die Verbindungselemente (5) im Durchgang (4) gegenseitig berühren.

10. Vorrichtung (1) nach einem der Ansprüche 1-9, **dadurch gekennzeichnet, dass** die Knochenfixationselemente (2) Knochenschrauben sind und der im Knochen verankerbare Teil (3) ein gewindeter Schraubenschaft ist, wobei die Knochenschraube einen Kopf (7) aufweist in welchem ein Durchgang (4) in Form eines U-förmigen Schlitzes realisiert ist, der mit einem als Kappe realisierten Blockierelement (6) derart verschliessbar ist, dass die Verschieblichkeit von sich im Schlitz befindlichen Verbindungselementen (5) blockierbar ist.

11. Vorrichtung (1) nach einem der Ansprüche 1 - 10, **dadurch gekennzeichnet, dass** die Oberfläche der flexiblen Verbindungselemente (5) eine Aufrauhung oder Mikrostrukturierung aufweist.

12. Vorrichtung (1) nach einem der Ansprüche 1-11, **dadurch gekennzeichnet, dass** die Oberfläche der flexiblen Verbindungselemente (5) eine Makrostrukturierung aufweist.

13. Vorrichtung (1) nach einem der Ansprüche 1-12, **dadurch gekennzeichnet, dass** die Oberfläche des Durchgangs (4) eine Aufrauhung oder Mikrostrukturierung aufweist.

## Claims

1. A bone fixation device (1) having at least two bone fixation elements (2) which are both provided with a portion (3) to be anchored on or in the bone and with a passage (4), whereby the device (1) comprises at least two longitudinal, flexible connecting members (5) which may be inserted in the passages (4) of two adjacent ones of a number of bone fixation elements (2) and fastened therein,
**characterized in that**
the number of connecting members (5) is four, five, six, or seven.

2. The device (1) as claimed in claim 1, **characterised in that** the bone fixation elements (2) are provided with a blocking element (6) by means of which the mobility of connecting members (5) inserted in the passage (4) relative to the bone fixation element (2) may optionally be fastened and released.

3. The device (1) as claimed in claim 1 or 2, **characterised in that** the bone fixation elements (2) are selected from among the following group: bone screws, bone retractors, and bone plates.

4. The device (1) as claimed in claims 1 to 3, **characterised in that** the passage (4) is a peripherally closed channel.

5. The device (1) as claimed in claims 1 to 3, **characterised in that** the passage (4) is a peripherally open channel, preferably in the form of a groove having a U-shaped profile.

6. The device (1) as claimed in any of the claims 1 to 5, **characterised in that** the connecting members (5) are preferably rod-like, i.e. provided with a round cross-sectional profile.

7. The device (1) as claimed in any of the claims 1 to 6, **characterised in that** the diameter of the passages (4) and the diameters of the connecting members (5) are adjusted to one another, so that the connecting members (5) may be received in the passages (4) in positive engagement.

8. The device (1) as claimed in any of the claims 1 to 7, **characterised in that** the cross section of the passages (4) is polyhedral, preferably square, rectangular, or triangular.

9. The device (1) as claimed in any of the claims 1 to 8, **characterised in that** the connecting members (5) inserted in the passage (4) are in contact with one another.

10. The device (1) as claimed in any of the claims 1 to 9, **characterised in that** the bone fixation elements (2) are bone screws and the portion (3) to be anchored in the bone is a threaded screw shank, the bone screw being provided with a head (7) in which a passage (4) in the form of a U-shaped groove is realised which may be closed by means of a blocking element (6) realised in the form of a cap, so that the displaceability of connecting members (5) placed in the groove can be inhibited.

11. The device (1) as claimed in any of the claims 1 to 10, **characterised in that** the surface of the flexible connecting members (5) is provided with a roughened or microstructured pattern.

12. The device (1) as claimed in any of the claims 1 to 11, **characterised in that** the surface of the flexible connecting members (5) is provided with a macrostructured pattern.

13. The device (1) as claimed in any of the claims 1 to 12, **characterised in that** the surface of the passage (4) is provided with a roughened or microstructured pattern.

## Revendications

1. Dispositif de fixation osseuse (1) comprenant au moins deux éléments de fixation osseuse (2), qui présentent tous deux une partie (3) pouvant être ancrée sur ou dans un os, et un passage (4), le dispositif (1) comprenant au moins deux éléments de liaison longitudinaux flexibles (5) pouvant être insérés dans les passages (4) de deux éléments de fixation osseuse adjacents (2) et pouvant y être bloqués,
**caractérisé en ce que**
le nombre d'éléments de liaison (5) est de quatre, cinq, six ou sept.

2. Dispositif (1) selon la revendication 1, **caractérisé en ce que** les éléments de fixation osseuse (2) présentent un élément de blocage (6) permettant au choix de bloquer et de libérer la mobilité des éléments de liaison (5) insérés dans le passage (4) par rapport à l'élément de fixation osseuse (2).

3. Dispositif selon la revendication 1 ou 2, **caractérisé en ce que** les éléments de fixation osseuse (2) sont choisis dans le groupe suivant : vis à os, crochets à os et plaques d'ostéosynthèse.

4. Dispositif selon l'une quelconque des revendications 1 à 3, **caractérisé en ce que** le passage (4) est un canal fermé au niveau de la périphérie.

5. Dispositif (1) selon l'une quelconque des revendications 1 à 3, **caractérisé en ce que** le passage (4) est un canal ouvert au niveau de la périphérie, de préférence sous la forme d'une rainure avec un profil en U.

6. Dispositif (1) selon l'une quelconque des revendications 1 à 5, **caractérisé en ce que** les éléments de liaison (5) se présentent sous la forme de barres de préférence avec un profil de section transversale rond.

7. Dispositif (1) selon l'une quelconque des revendications 1 à 6, **caractérisé en ce que** la coupe droite des passages (4) et les coupes droites des éléments de liaison (5) sont adaptées les unes aux autres de telle sorte que les éléments de liaison (5) peuvent être logés par emboîtement dans les passages (4).

8. Dispositif (1) selon l'une quelconque des revendications 1 à 7, **caractérisé en ce que** la coupe droite des passages (4) est réalisée sous la forme d'un polyèdre, de préférence sous la forme d'un carré, d'un rectangle ou d'un triangle.

9. Dispositif (1) selon l'une quelconque des revendications 1 à 8, **caractérisé en ce que** les éléments de liaison (5) sont mutuellement en contact dans le passage (4).

10. Dispositif (1) selon l'une quelconque des revendications 1 à 9, **caractérisé en ce que** les éléments de fixation osseuse (2) sont des vis à os et la partie (3) pouvant être ancrée dans l'os est une tige de vis filetée, la vis à os présentant une tête (7) dans laquelle a été réalisé un passage (4) sous la forme d'une fente en U, qui peut être fermée à l'aide d'un élément de blocage (6) réalisé sous la forme d'un capuchon, de telle sorte que le coulissage des éléments de liaison (5) se trouvant dans la fente peut être bloqué.

11. Dispositif (1) selon l'une quelconque des revendications 1 à 10, **caractérisé en ce que** la surface des éléments de liaison flexibles (5) présente une rugosité ou une microtexture.

12. Dispositif (1) selon l'une quelconque des revendications 1 à 11, **caractérisé en ce que** la surface des éléments de liaison flexibles (5) présente une macrotexture.

13. Dispositif (1) selon l'une quelconque des revendications 1 à 12, **caractérisé en ce que** la surface du passage (4) présente une rugosité ou une microtexture.
